(19) Europäisches Patentamt
European Patent Office
Office européen des brevets

(11) Publication number: **0 423 524 A2**

(12) **EUROPEAN PATENT APPLICATION**

(21) Application number: **90118675.9**

(22) Date of filing: **28.09.90**

(51) Int. Cl.5: **C07D 233/60**, C07D 295/10, C07D 249/08, A61K 31/415, A61K 31/495, A61K 31/445, A61K 31/40, A61K 31/41

(30) Priority: **28.09.89 JP 253934/89**

(43) Date of publication of application: **24.04.91 Bulletin 91/17**

(84) Designated Contracting States: **DE FR GB**

(71) Applicant: **MARUHO Co., Ltd.**
**6-24, Nakatsu 1-chome, Kita-ku**
**Osaka 531(JP)**

(72) Inventor: **Okada, Shigeya**
**17-1-501, Higashinakaburi 2-chome**
**Hirakata-shi Osaka(JP)**
Inventor: **Miyagawa, Hiromasa**
**55-9, Shimogawara-cho Fukakusa**
**Fushimi-ku Kyoto(JP)**
Inventor: **Sugimoto, Seiichi**
**4-10-30-613, Sekime**
**Joto-ku Osaka(JP)**
Inventor: **Yamada, Mitsuo**
**16-3, Tomogaoka 2-chome**
**Sanda-shi Hyogo-ken(JP)**
Inventor: **Nakamura, Munehiko**
**7-4, Tenjin 4-chome**
**Nagaokakyo-shi Kyoto(JP)**

(74) Representative: **Patentanwälte Beetz sen. - Beetz jun. Timpe - Siegfried - Schmitt-Fumian- Mayr**
**Steinsdorfstrasse 10**
**W-8000 München 22(DE)**

(54) **Propiophenone derivatives and their preparation and pharmaceutical use.**

(57) The present invention relates to compounds represented by the general formula I

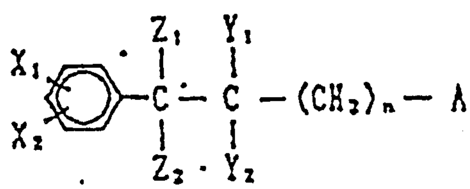

(I),

in which

- $X_1$, $X_2$ represent hydrogen, $C_{1-4}$-alky phenyl, halogen, $C_{1-4}$-alkoxy or dialkylamino with $C_{1-4}$-alkyl groups, or $X_1$ and $X_2$ together represent phenylene or indane-2,3-diyl,
- $Z_1$, $Z_2$ represent hydrogen or hydroxy, or together represent oxygen or $-O(CH_2)_2O-$,
- $Y_1$, $Y_2$ represent hydrogen, $C_{1-3}$-alkyl or phenyl,
- A represents a group derived from a cyclic amine, and
- n represents 0, 1 or 2,

in the form of the free base or in the form of addition salts, preferably pharmaceutically acceptable salts, to methods for their preparation, and to their pharmaceutical use as central muscle relaxants and anticonvulsives.

EP 0 423 524 A2

## PROPIOPHENONE DERIVATIVES AND THEIR PREPARATION AND PHARMACEUTICAL USE

The present invention relates to a class of novel propiophenon derivatives and a process for preparing them. The invention further relates to pharmaceutical compositions, particularly with central muscle relaxant and anticonvulsive activity, containing such derivatives, for specifically removing the tone of skeletal muscles without unconsciousness through action to the central nervous system.

As a general rule, central muscle relaxants are used for treating convulsive diseases of skeletal muscles or muscle spasms accompanied by trauma and inflammation. Anaesthetcis, hypnotics or tranquilizers etc. generally have muscular relaxant action. These drugs, however, cannot be employed for central muscle relaxation, because other central actions are strong. In principle, a central muscle relaxant is required to specifically moderate the tone of skeletal muscles without unconsciousness. Drugs such as chlorphenesin carbamate, eperisone hydrochloride or baclophene etc. have been used in the past as central muscle relaxants.

However, central muscle relaxants such as chlorphenesin carbamate, eperisone hydrochloride or baclophene etc. have inferior pharmacological activity, and thus a sufficient effect cannot be expected. Accordingly, a central muscle relaxant having a high pharmacological effect has been required.

Eperisone hydrochloride has the structure shown by the following formula:

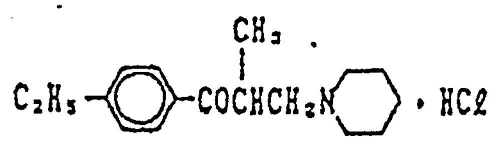

As compounds similar to eperisone hydrochloride, JP-A-54-125630 discloses the following compounds: Compounds made by substituting isobutyl or phenyl for the methyl group of the eperisone molecule, and compounds made by substituting another cyclic amino group for the piperidino group of the eperisone molecule. These compounds, however, show antiallergic action. No mention has been made with respect to the pharmacological effect of central muscle relaxant action.

Some derivatives made by substituting the imidazole-1-yl for piperidino of the eperisone molecule are described as antispasmodic in JP-A-55-19294.

According to experiments in connection with the present invention, it has become evident that especially excellent pharmacological effects could not be found with most of the compounds mentioned in this publication.

Accordingly, it is the object of the present invention to provide new compounds having an excellent central muscular relaxant and anticonvulsive action, and a process for their preparation.

The above object is achieved according to the claims. The dependent claims relate to preferred embodiments.

The compounds of the present invention are represented by the general formula I

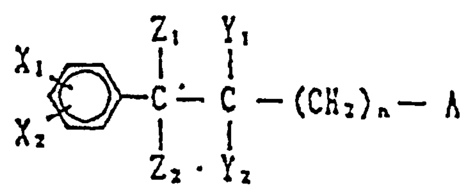 (I),

in which

- $X_1$, $X_2$, being the same or different, represent a hydrogen atom, $C_{1-4}$-alkyl, phenyl, halogen, $C_{1-4}$-alkoxy or dialkylamino with $C_{1-4}$-alkyl groups, or $X_1$ and $X_2$ together represent phenylene or indane-2,3-diyl,
- $Z_1$, $Z_2$, being the same or different, represent a hydrogen atom or hydroxy, or together represent oxygen or $-O(CH_2)_2O-$,
- $Y_1$, $Y_2$, being the same or different, represent a hydrogen atom, $C_{1-3}$-alkyl or phenyl,

A represents a group derived from a cyclic amine, and

n represents 0, 1 or 2,

in the form of the free base or in the form of addition salts, preferably pharmaceutically acceptable salts.

The salts with pharmacologically permissible acids are formed e.g. with inorganic acids such as hydrochloric acid, nitric acid, sulfuric acid, phosphoric acid and the like, or with organic acids, such as acetic acid, maleic acid, and so forth.

Preferred compounds of the general formula I are those wherein $X_1$, $X_2$, being the same or different, represent a hydrogen atom, $C_{1-4}$-alkyl or halogen, respectively, or together represent phenylene.

According to further preferred embodiments, $X_1$ represents 4-ethyl, and $X_2$ represents a hydrogen atom, or $X_1$, $X_2$ together represent 3,4-dimethyl or phenylene.

In case an alkyl is introduced as $X_1$, $X_2$, the compounds possess an enhanced inhibitory effect on the motor coordination, anti-nicotinic action and anti-strychnine action, etc. In particular, in those cases where $C_{1-3}$-alkyl groups, that is, methyl, ethyl or propyl, are introduced, enhanced action is provided. Furthermore, the introduction of ethyl provides the strongest action.

In the compounds of the general formula I, preferably one of $Y_1$ and $Y_2$ represents methyl, and the other represents a hydrogen atom.

In case an alkyl is introduced in at least one of the positions of $Y_1$, $Y_2$, an enhanced inhibitory effect on the motor coordination and antinicotinic action is obtained.

And, in the compounds of the general formula I, if n is ≥ 1 and preferably n is equal to 1, and an alkyl is introduced in at least one of the positions $Y_1$, $Y_2$, frequently an inhibitory effect on the motor coordination is obtained.

In the compounds of the general formula I, preferably A is a piperidino, pyrrolidine-1-yl or imidazole-1-yl, which is most preferred.

In case imidazole-1-yl is introduced as A, an enhanced inhibitory effect on the motor coordination and anti-nicotinic action is obtained. Then, for both of these activities, long-term action is found, and simultaneously anti-strychnine action is generated, and an excellent central muscle relaxant action is expected.

In the compounds of the general formula I, preferably $Z_1$, $Z_2$ together represent oxygen, and one of $Y_1$ and $Y_2$ represents methyl, and the other is a hydrogen atom.

Examples for compounds of the general formula I having excellent central muscle relaxant and anticonvulsive activity are compounds of the following formulae:

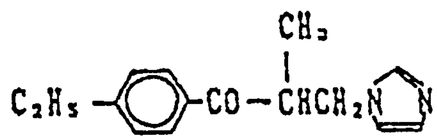

(II)

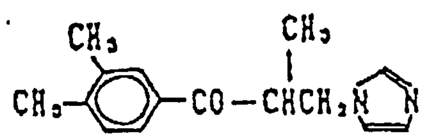

(III)

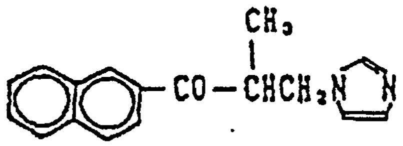

(IV)

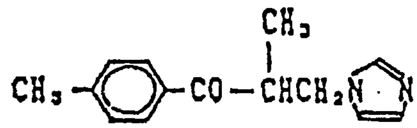

(V)

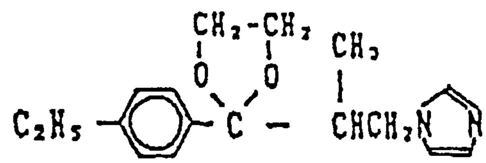

(VI)

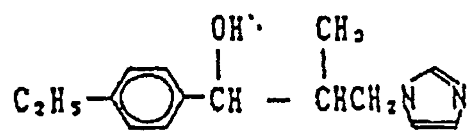

(VII)

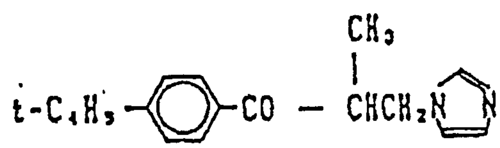

(VIII)

The main process for preparing the compounds of the general formula I of the present invention will hereinafter be described in detail.

The method for preparing the compounds represented by the general formula I is characterized by the following measures:

(A) For preparing compounds of the general formula X,

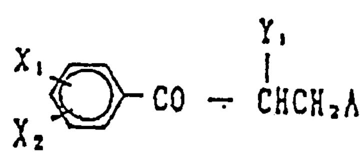

(X),

wherein $X_1$, $X_2$, $Y_1$ and A are defined as above,

a compound of the general formula IX,

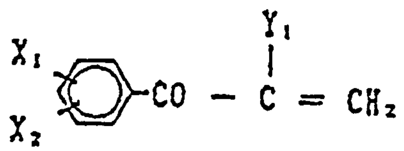

(IX),

wherein $X_1$, $X_2$ and $Y_1$ are defined as above,

is caused to react with a cyclic amine or a reactive derivative thereof, preferably in a solvent such as methanol, etc., and preferably under reflux.

A preferred compound of formula X is a compound wherein $X_1$ is 4-ethyl, $X_2$ is hydrogen, $Y_1$ is methyl, and A is imidazol-1-yl.

In case the starting compound IX is not commercially available, it may be obtained in a usual manner, e.g. by introducing suitable substituents into analogous compounds, or by means of intramolecular condensation. In order to purify the respective compounds, the solvent may be evaporated, an then, the residue may be extracted with a solvent, such as chloroform, etc., or may be purified by column chromatography.
(B) For preparing compounds of the general formula XII,

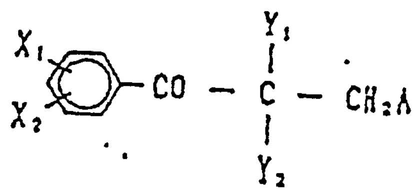

(XII),

wherein $X_1$, $X_2$, $Y_1$, $Y_2$ and A are defined as above,

a compound of the general formula XI,

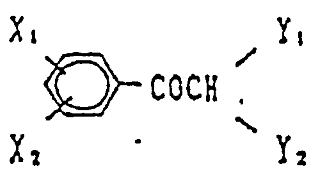

(XI),

wherein $X_1$, $X_2$, $Y_1$ and $Y_2$ are defined as above,

is caused to react with paraformaldehyde or formalin and the respective cyclic amine or a reactive derivative thereof or a mineral acid salt thereof, particularly the hydrochloride.

The reaction is preferably carried out in a solvent such as methanol, ethanol, isopropanol, etc., preferably under reflux.

In case the starting compound XI is not commercially available, it may be obtained in a usual manner, e.g. by introducing suitable substituents into analogous compounds, or by means of intramolecular condensation.

In order to purify the respective compounds, the solvent may be evaporated, and then, the residue may be extracted with a solvent, such as chloroform, etc., or may be purified by column chromatography.
(C) For preparing compounds of the general formula XIV,

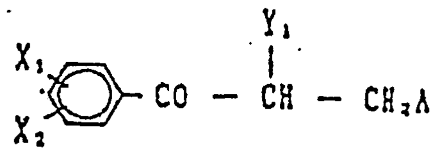

(XIV),

wherein $X_1$, $X_2$, $Y_1$ and A are defined as above,

a methoiodide compound of the general formula XIII,

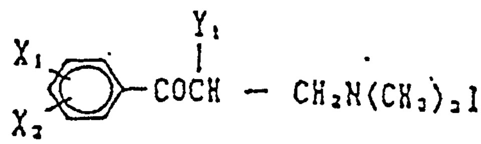

(XIII),

wherein $X_1$, $X_2$ and $Y_1$ are defined as above,

is caused to react with a cyclic amine or a reactive derivative thereof.

Preferred compounds of formula XIV are those wherein

- $X_1$ is 4-methyl, $X_2$ is 3-methyl, $Y_1$ is methyl, and A is imidazole-1-yl, or
- $X_1$,$X_2$ together represent phenylene, $Y_1$ is methyl, and A is imidazole-1-yl, or
- $X_1$ is 4-methyl, $X_2$ is a hydrogen atom, $Y_1$ is methyl, and A is imidazole-1-yl, or
- $X_1$ is tert-butyl, $X_2$ is a hydrogen atom, $Y_1$ is methyl, and A is imidazole-1-yl.

In case the starting compound XIII is not commercially available, it may be obtained in a usual manner, e.g. by introducing suitable substituents into analogous compounds, or by means of intramolecular condensation.

In order to purify the respective compounds, the solvent may be evaporated, and then, the residue may be extracted with a solvent, such as chloroform, etc., or may be purified by column chromatography.

(D) For preparing compounds of the general formula XVI,

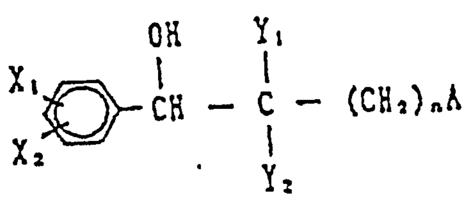

(XVI),

wherein $X_1$, $X_2$, $Y_1$, $Y_2$, A and n are defined as above,

a compound of the general formula XV,

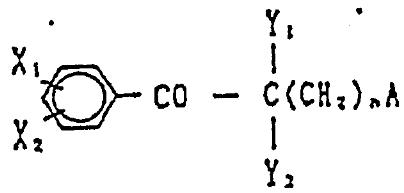

(XV),

wherein $X_1$, $X_2$, $Y_1$, $Y_2$, A and n are defined as above,

is reduced by means of a reducing agent.

A preferred compound of formula XVI is a compound wherein $X_1$ is 4-ethyl, $X_2$ is a hydrogen atom, $Y_1$ is methyl, $Y_2$ is a hydrogen atom, A is imidazole-1-yl, and n is 1.
(E) For preparing compounds of the general formula XVII,

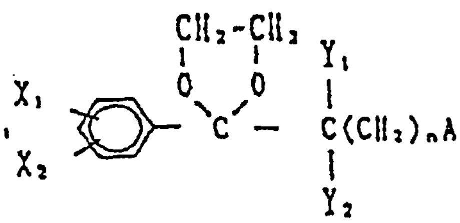

(XVII),

wherein $X_1$, $X_2$, $Y_1$, $Y_2$, A and n are defined as above,

a compound of the above-defined formula XV

is caused to react with ethylene glycol.

A preferred compound of formula XVII is a compound wherein $X_1$ is 4-ethyl, $X_2$ is a hydrogen atom, $Y_1$ is methyl, $Y_2$ is a hydrogen atom, A is imidazole-1-yl, and n is 1.

In case the starting compound XV is not commercially available, it may be obtained in a usual manner, e.g. by introducing suitable substituents into analogous compounds, or by means of intramolecular condensation.

In order to purify the respective compounds, the solvent may be evaporated, and then, the residue may be extracted with a solvent, such as chloroform, etc., or may be purified by column chromatography.
(F) For preparing compounds of the general formula XIX,

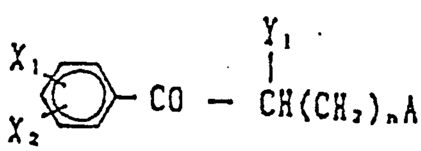

(XIX),

wherein $X_1$, $X_2$, $Y_1$, A and n are defined as above,

a compound of the general formula XVIII,

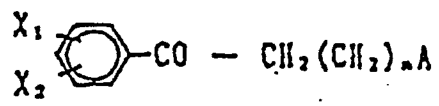

(XVIII),

wherein $X_1$, $X_2$, A and n are defined as above,

is caused to react with sodium hydride and an alkyl halide.

A preferred compound XIX is a compound wherein $X_1$ is 4-ethyl, $X_2$ is a hydrogen atom, $Y_1$ is methyl, A is imidazole-1-yl, and n is 0.

In case the starting compound XVIII is not commercially available, it may be obtained in a usual manner, e.g. by introducing suitable substituents into analogous compounds, or by means of intramolecular condensation.

In order to purify the respective compounds, the solvent may be evaporated, and then, the residue may be extracted with a solvent, such as chloroform, etc., or may be purified by column chromatography.

For preparing salts of the compounds of formula I, the compound, which may have been obtained in solid and/or crystalline form, is first dissolved in a suitable solvent, such as ethanol, and a suitable acid is added, if not already comprised in the solvent used, to make the corresponding acid addition salt, preferably the hydrochloride, obtained with use of hydrochloric acid. The acid addition salt is then recrystallized in a usual manner.

For pharmaceutical applications, pharmaceutically acceptable salts are used, e.g. the hydrochlorid, phosphate, acetate, maleate, etc. The compounds of formulae I to VIII as defined above possess central muscle relaxant and anticonvulsive activity.

In accordance therewith, the pharmaceutical compositions of the present invention are characterized in that they comprise one or more of the compounds of formulae I to VIII as active ingredients, salts thereof being pharmaceutically acceptable.

The compounds of formulae I to VIII and their pharmaceutically acceptable salts may be used as such as drugs.

For preparing pharmaceutical formulations, the active ingredients are mixed with appropriate, usual additives, such as diluents, carriers, stabilizers, solvents, emulsifiers, colorants, etc.

The dosage form used depends upon the specific purpose. Various dosage forms can be applied as for conventional treatment methods with known central muscle relaxants and anticonvulsives without any specific limitation. The compounds and compositions are usually administered orally. Suitable pharmaceutical preparation forms are tablets, capsules, powders, syrups, suspensions, emulsions, pills, tapes and the like.

Diluents and excipients for pharmaceutical preparations are those routinely used in this field, and the following can be used as required according to the dosage form: For example, starch such as potato, corn, rice, barley/wheat starch or the like, cellulose lactose, sucrose, mannitol, sorbitol, gelatin, gum arabic, tragacanth, polyvinyl pyrrolidone, carboxymethyl cellulose, sodium carboxymethylcellulose, calcium carboxymethylcellulose, talc, magnesium stearate, calcium stearate, polyethylene glycol, polysorbate, glycerin, cacao butter, macrogol, ethanol, and the like.

In case the compounds or compositions of the present invention are administered using an oral dosage form a daily dose of 10 to 1000 mg is thought adequate for adults, according to the symptoms.

A divided administration is also possible at appropriate time intervals. The above-mentioned dose, however, is an example for the average adult. For instance, according to the weight and disease of the treated subject, these doses may be changed as required, or it may be departed therefrom.

## Pharmacological activity

The compounds and compositions in accordance with the present invention have an excellent central muscle relaxation and anticonvulsive action. The voluntary movement in case of an injury of the extrapyramidal system is mainly controlled, or the postural and physical dysequilibrium is remitted as mentioned above. That is to say, the compounds and compositions are extremely useful for the therapy of painful muscle spasms such as cerebral apoplexy sequela, cerebral palsy, spastic spinal paralysis, spastic paralysis caused by head injury, and lumbago/dorsalgia, cervicodynia/shoulder/arm pains, etc. It is also considered that the compounds and compositions in accordance with the present invention can be used for the therapy of Parkinson's syndrome. A specific advantage is the possibility of oral administration.

In the following, the invention will be further explained by way of examples.

## I. Preparation Examples

The preparation of compounds of the present invention is described in detail as follows:

(1) Preparation of N,N'-bis[2-(4-ethylbenzoyl)-propyl]-piperazine dihydrochloride (compound 1):

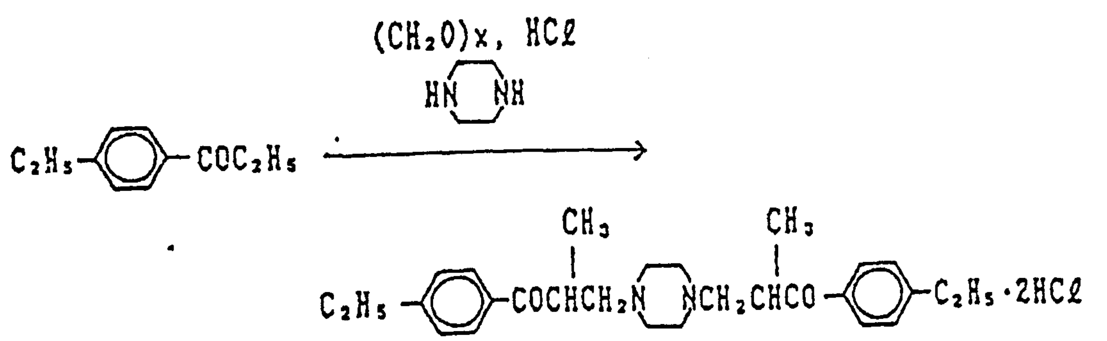


A mixture of 12 g (0.074 mol) of 4-ethyl-propiophenone, 3 g of paraformaldehyde, 2.7 g (0.031 mol) of piperazine, 8 ml of hydrochloric acid and 20 ml of ethanol was refluxed overnight, and then acetone was added to the reaction solution. The precipitate was filtered off and recrystallized from water/ethanol. White crystals.

| | |
|---|---|
| Yield: | 1.0 g ( 6 %) |
| Melting point: | Decomposition above 156 °C |

Elemental analysis:

| | C (%) | H (%) | N (%) |
|---|---|---|---|
| Found: | 65.66 | 7.97 | 4.99 |
| Calculated: | 66.26 | 7.94 | 5.52. |

(2) Preparation of 1-(4-ethylphenyl)-3-(1H-imidazol-1-yl)-2-methylpropan-1-one (compound 2):

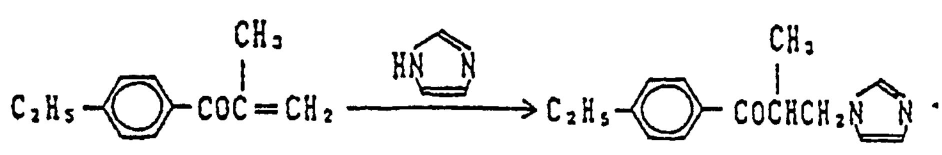

A mixture of 7.5 g (0.043 mol) of 1-(4-ethylphenyl)-2-methyl-2-propen-1-one, 3.0 g (0.044 mol) of

imidazole and 50 ml of methanol was refluxed for 4 hours, and then the methanol was evaporated. Next, the residue was extracted with chloroform and purified by column chromatography.
White lump.

| | | | |
|---|---|---|---|
| Yield: | 4.5 g (43 %) | | |
| Melting point: | 45.8 - 47.0 °C | | |
| Elemental analysis: | | | |
| | C (%) | H (%) | N (%) |
| Found: | 74.21 | 7.47 | 11.59 |
| Calculated: | 74.35 | 7.49 | 11.56. |

(3) Preparation of 1-(4-chlorobenzoyl)-3-(1H-imidazol-1-yl)-2-methylpropan-1-one hydrochloride (compound 3):

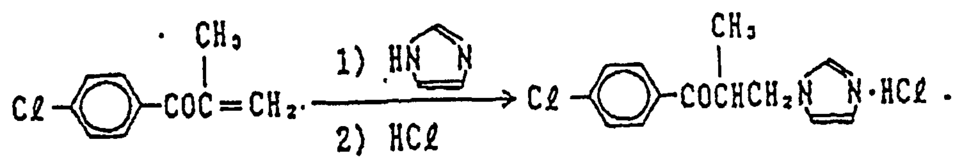


A mixture of 9 g (0.05 mol) of 1-(4-chlorophenyl)-2-methyl-2-propen-1-one, 3.4 g (0.05 mol) of imidazole and 50 ml of methanol was refluxed for 4 hours, and then the methanol was evaporated. Next, the residue was extracted with chloroform, concentrated in vacuo and dissolved in ethanol. 10 ml of hydrochloric acid were added to convert it into the hydrochloride which was recrystallized from isopropanol/ether.
White crystals.

| | | | |
|---|---|---|---|
| Yield: | 9.8 g (68 %) | | |
| Melting point: | 144.4 - 146.0 °C. | | |
| Elemental analysis: | | | |
| | C (%) | H (%) | N (%) |
| Found: | 55.03 | 5.09 | 9.27 |
| Calculated: | 54.75 | 4.95 | 9.82. |

(4) Preparation of 2-ethyl-1-(4-ethylphenyl)-3-piperidinopropan-1-onehydrochloride (compound 4):

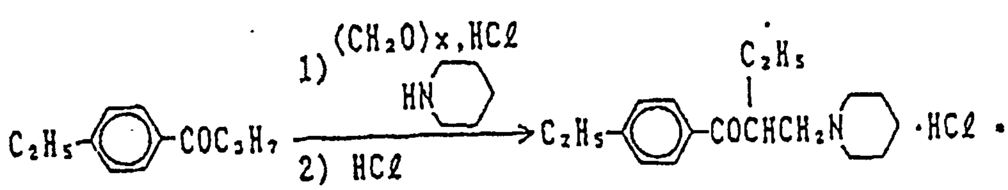


A mixture of 9.0 g (0.051 mol) of 1-(4-ethylphenyl)-butan-1-one, 1.6 g of paraformaldehyde, 4.3 g (0.051

mol) of piperidine, 5 ml of hydrochloric acid and 30 ml of isopropanol was refluxed overnight, and then the isopropanol was evaporated. 50 ml of water were added to the residue, which was washed with n-hexane, neutralized with alkali, and extracted with chloroform. The chloroform was evaporated, and the residue was dissolved in ethanol. 5 ml of hydrochloric acid were added to convert it into the hydrochloride which was recrystallized from acetone.
White crystals.

Yield: 5.4 g (34 %)
Melting point: 160.4 - 162.0 °C
Elemental analysis:

| | C (%) | H (%) | N (%) |
|---|---|---|---|
| Found: | 69.77 | 9.11 | 4.52 |
| Calculated: | 69.36 | 9.09 | 4.44. |

(5) Preparation of 1-(4-ethylphenyl)-2-isopropyl-3-piperidinopropan-1-one (compound 5):

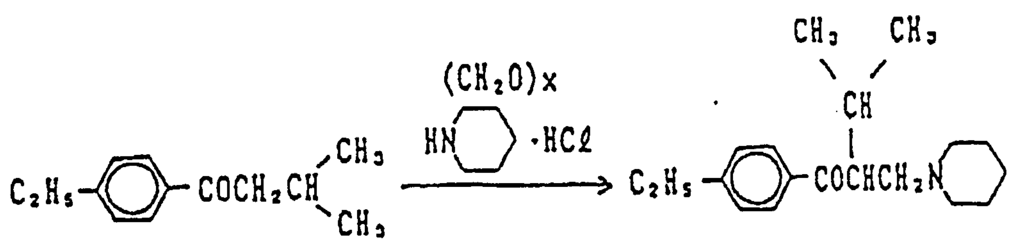


A mixture of 19.0 g (0.10 mol) of 1-(4-ethylphenyl)-3-methylbutan-1-one,15.0 g of paraformaldehyde, 12.1 g (0.10 mol) of piperidine hydrochloride, and 30 ml of isopropanol was refluxed overnight, and then the solvent was evaporated. Water was added to the residue, which was washed with ether, neutralized with alkali, and extracted with ether. The ether was evaporated, and then, the residue was purified by column chromatography. Yellow oil.

Yield: 6.5 g (23 %)
Elemental analysis:

| | C (%) | H (%) | N (%) |
|---|---|---|---|
| Found: | 79.39 | 10.17 | 4.87 |
| Calculated: | 78.40 | 10.15 | 4.82. |

(6) Preparation of 1-(4-ethylphenyl)-2,2-dimethyl-3-piperidinopropan-1-one (compound 6):

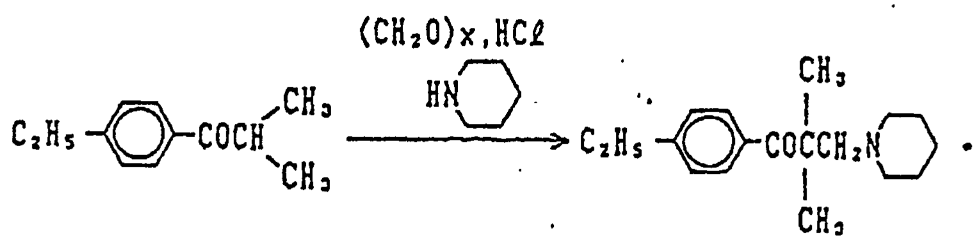


A mixture of 18.0 g (0.102 mol) of 1-(4-ethylphenyl)-2-methylpropan-1-one, 15.0 g of paraformaldehyde, 8.5 g (0.10 mol) of piperidine and 10 ml of hydrochloric acid was stirred overnight with heating in an oil bath set to 110 °C. After cooling, water was added thereto. After washing with ether and neutralization with alkali, it was extracted with ether. The ether was then evaporated, and the residue was purified by column chromatography.
Yellow oil.

| | | | |
|---|---|---|---|
| Yield: | 0.8 g (3 %) | | |
| Elemental analysis: | | | |
| | C (%) | H (%) | N (%) |
| Found: | 79.08 | 9.95 | 5.12 |
| Calculated: | 79.08 | 10.06 | 5.11. |

(7) Preparation of 1-(4-ethylphenyl)-2-phenyl-3-piperidinopropan-1-one hydrochloride (compound 7):

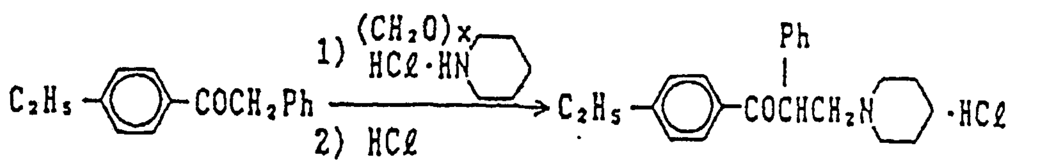


A mixture of 22.2 g (0.100 mol) of 1-(4-ethylphenyl)-2-phenylethan-1-one, 5.0 g of paraformaldehyde, 9.0 g (0.074 mol) of piperidine hydrochloride, and 30 ml of isopropanol was refluxed overnight, and then the solvent was evaporated. Water was added to the residue, which was washed with ether, neutralized with alkali, and extracted with ether. The ether was evaporated, and then the residue was dissolved in ethanol. 100 ml of hydrochloric acid was added to convert it into the hydrochloride which was re-crystallized from ethanol/ether.
White crystals.

| | | | |
|---|---|---|---|
| Yield: | 11.8 g (45 %) | | |
| Melting point: | 146.0 - 147.0 °C | | |
| Elemental analysis: | C (%) | H (%) | N (%) |
| Found: | 73.83 | 7.89 | 3.91 |
| Calculated: | 73.09 | 7.86 | 4.01. |

(8) Preparation of 3-(1H-imidazol-1-yl)-2-methyl-1-phenyl-propan-1-one (compound 8):

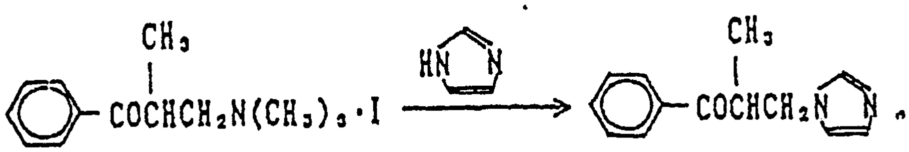

A mixture of 15.0 g (0.045 mol) of 2-methyl-3-dimethyl-amino-1-phenylpropan-1-one methoiodide, 3.0 g (0.045 mol) of imidazole and 50 ml of ethanol was refluxed for 6 hours, and then the ethanol was evaporated. Water was added to the residue, which was extracted with ether. Next the ether was evaporated, and the residue was purified by column chromatography.
White crystals.

| | | | |
|---|---|---|---|
| Yield: | 3.3 g (34 %) | | |
| Melting point: | 76.8 - 77.3 °C | | |
| Elemental analysis: | | | |
| | C (%) | H (%) | N (%) |
| Found: | 72.87 | 6.59 | 13.07 |
| Calculated: | 72.88 | 6.66 | 13.05. |

(9) Preparation of 3-(1H-imidazol-1-yl)-2-methyl-1-(4-n-propylphenyl)-propan-1-one (compound 9):

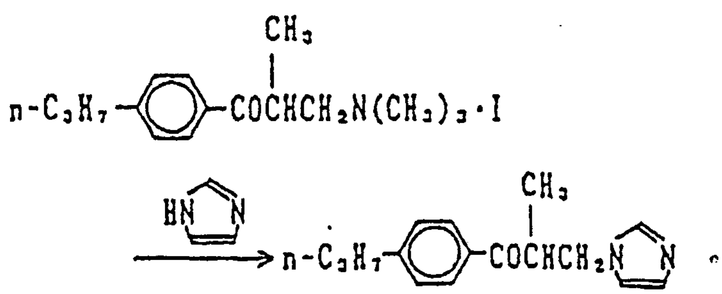

A mixture of 20.0 g (0.053 mol) of 2-methyl-3-dimethylamino-1-(4-n-propylphenyl)-propan-1-one methoiodide, 2.9 g (0.043 mol) of imidazole and 100 ml of methanol was refluxed for 3 hours, and then the methanol was evaporated. Water was added to the residue, which was extracted with ether. Next the ether was evaporated, and the residue was purified by column chromatography.
White crystals.

| | | | |
|---|---|---|---|
| Yield: | 4.4 g (40 %) | | |
| Melting point: | 49.5 - 50.5 °C | | |
| Elemental analysis: | C (%) | H (%) | N (%) |
| Found: | 74.95 | 7.86 | 10.93 |
| Calculated: | 74.47 | 7.81 | 10.81. |

(10) Preparation of 3-(1H-imidazol-1-yl)-1-(4-isopropylphenyl)-2-methylpropan-1-one (compound 10):

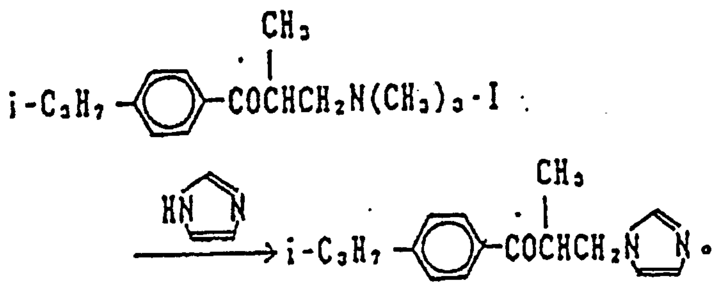

A mixture of 20.0 g (0.053 mol) of 1-(4-isopropylphenyl)-2-methyl-3-dimethylaminopropan-1-one methoiodide, 2.9 g (0.043 mol) of imidazole and 100 ml of methanol was refluxed for 3 hours, and then the methanol was evaporated. Water was added to the residue, which was extracted with ether. Next the ether was evaporated, and the residue was purified by column chromatography.
White crystals.

| | | | |
|---|---|---|---|
| Yield: | 5.6 g (51 %) | | |
| Melting point: | 73.2 - 74.2 °C | | |
| Elemental analysis: | C (%) | H (%) | N (%) |
| Found: | 74.75 | 7.86 | 10.93 |
| Calculated: | 74.85 | 7.97 | 10.86. |

(11) Preparation of 1-(4-t-butylphenyl)-3-(1H-imidazol-1-yl)-2-methylpropan-1-one (compound 11):

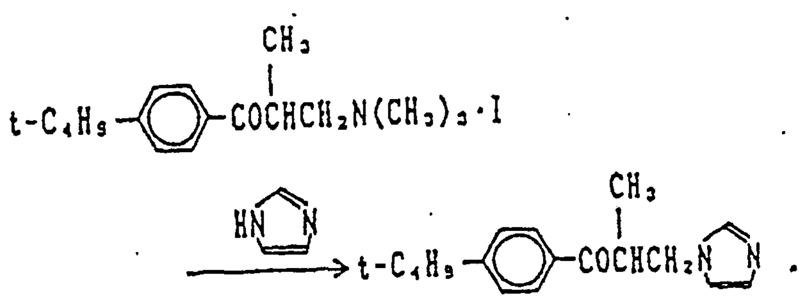

A mixture of 21.0 g (0.054 mol) of 1-(4-butylphenyl)-2-methyl-3-dimethylaminopropan-1-one methoiodide, 2,9 g (0.043 mol) of imidazole and 100 ml of methanol was refluxed for 3 hours, and then the methanol was evaporated. Water was added to the residue, which was extracted with ether. Next the ether was evaporated, and the residue was purified by column chromatography.
White lump.

| | | | |
|---|---|---|---|
| Yield: | 6.2 g (53 %) | | |
| Melting point: | 65.0 - 67.6 °C | | |
| Elemental analysis: | | | |
| | C (%) | H (%) | N (%) |
| Found: | 75.52 | 8.20 | 10.36 |
| Calculated: | 75.46 | 8.19 | 10.36. |

(12) Preparation of 1-(4-biphenyl)-3-(1H-imidazol-1-yl)-2-methylpropan-1-one (compound 12):

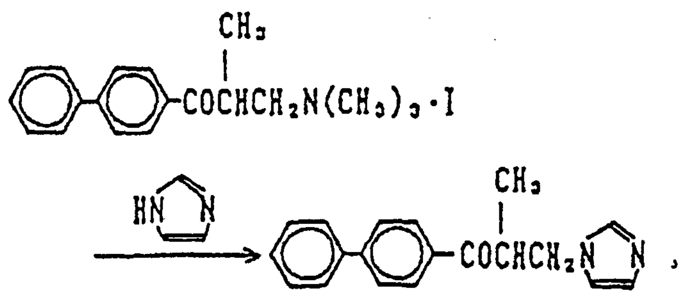

A mixture of 22.0 g (0.054 mol) of 1-(4-biphenyl)-2-methyl-3-dimethylaminopropan-1-one methoiodide, 2.9 g (0.043 mol) of imidazole and 100 ml of methanol was refluxed for 3 hours, and then the methanol was evaporated. Water was added to the residue, which was extracted with ether. Next the ether was evaporated, and the residue was purified by column chromatography.
White crystals.

| | | | |
|---|---|---|---|
| Yield: | 8.0 g (64 %) | | |
| Melting point: | 158.0 - 158.3 °C | | |
| Elemental analysis: | | | |
| | C (%) | H (%) | N (%) |
| Found: | 78.59 | 6.25 | 9.65 |
| Calculated: | 78.74 | 6.27 | 10.05. |

(13) Preparation of 3-(1H-imidazol-1-yl)-2-methyl-1-(2,4-dimethylphenyl)-propan-1-one hydrochloride (compound 13):

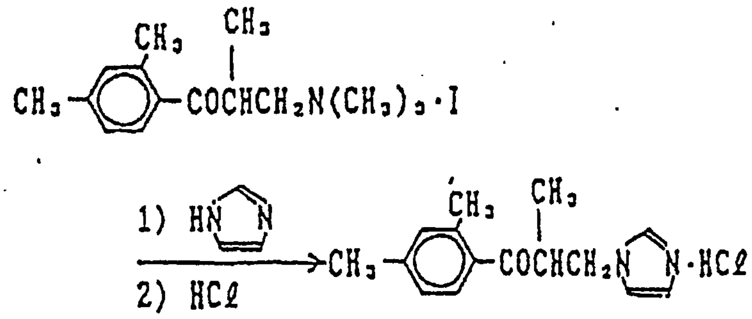


A mixture of 29.0 g (0.080 mol) of 2-methyl-3-dimethylamino-1-(2,4-dimethylphenyl)-propan-1-one methoiodide, 4.9 g (0.072 mol) of imidazole and 100 ml of methanol was refluxed for 3 hours, and then the methanol was evaporated. Water was added to the residue, which was extracted with ether. Next the ether was evaporated, and the residue was purified by column chromatography. Then, a 0.1N ethanol solution of hydrochloric acid was added to convert it into the hydrochloride which was crystallized from ether.
White crystals.

| | | | |
|---|---|---|---|
| Yield: | 4.1 g (53 %) | | |
| Melting point: | 133.6 - 135.2 °C | | |
| Elemental analysis: | C (%) | H (%) | N (%) |
| Found: | 64.63 | 6.87 | 10.05 |
| Calculated: | 64.52 | 6.86 | 10.09. |

(14) Preparation of 3-(1H-imidazol-1-yl)-2-methyl-1-(3,4-dimethylphenyl)-propan-1-one (compound 14):

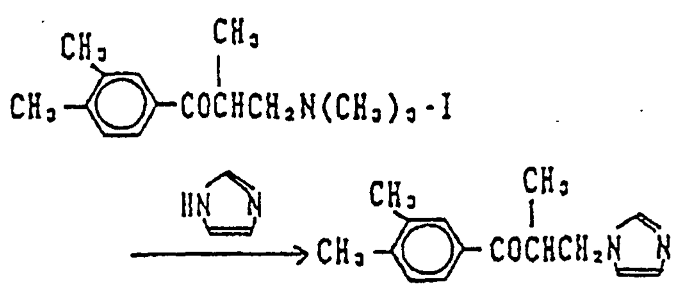

A mixture of 19.5 g (0.054 mol) of 2-methyl-3-dimethylamino-1-(3,4-dimethylphenyl)-propan-1-one methoiodide, 3.3 g (0.049 mol) of imidazole and 100 ml of methanol was refluxed for 3 hours, and then the methanol was evaporated. Water was added to the residue, which was extracted with ether. Next the ether was evaporated, and the residue was purified by column chromatography.
White crystals.

Yield: 8.1 g (34 %)

Melting point: 87.0 - 88.0 °C

Elemental analysis:

| | C (%) | H (%) | N (%) |
|---|---|---|---|
| Found: | 74.35 | 7.49 | 11.56 |
| Calculated: | 74.55 | 7.49 | 11.72. |

(15) Preparation of 3-(1H-imidazol-1-yl)-2-methyl-1-(2-naphthyl)-propan-1-one (compound 15):

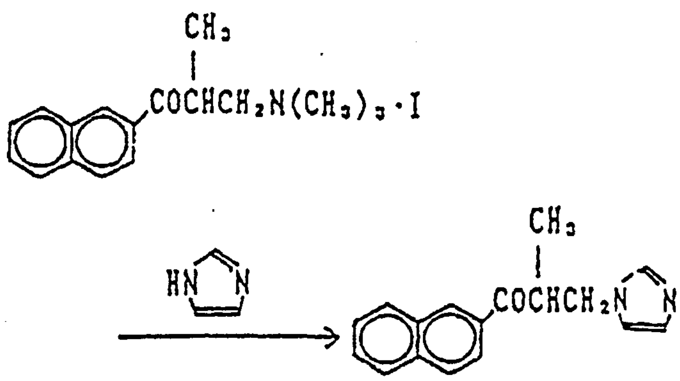

A mixture of 0.7 g (0.054 mol) of 2-methyl-3-dimethylamino-1-(2-naphthyl)-propan-1-one-methoiodide, 3.3 g (0.049 mol) of imidazole and 100 ml of methanol was refluxed for 3 hours, and then the methanol was evaporated. Water was added to the residue, which was extracted with ether. Next the ether was evaporated, and the residue was purified by column chromatography.
White crystals.

Yield: 6.7 g (52 %)

Melting point: 117.0 - 118.0 °C

Elemental analysis:

| | C (%) | H (%) | N (%) |
|---|---|---|---|
| Found: | 77.25 | 6.10 | 10.60 |
| Calculated: | 77.55 | 6.14 | 10.79. |

(16) Preparation of 3-(1H-imidazol-1-yl)-2-methyl-1-(4-methylphenyl)-propan-1-one (compound 16):

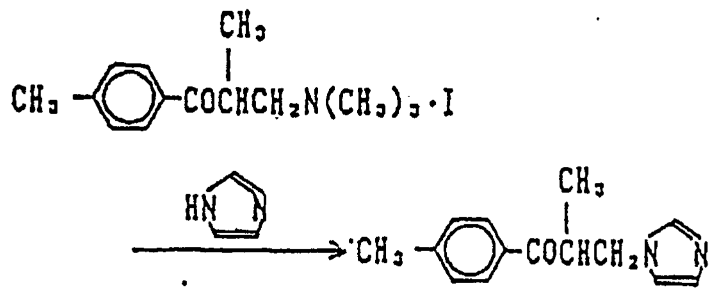

A mixture of 18.8 g (0.054 mol) of 2-methyl-3-dimethylamino-1-(4-methylphenyl)-propan-1-one methoiodide, 3.3 g (0.049 mol) of imidazole and 100 ml of methanol was refluxed for 3 hours, and then the methanol was evaporated. Water was added to the residue, which was extracted with ether. Next the ether was evaporated, and the residue was purified by column chromatography.
White crystals.

| | | | |
|---|---|---|---|
| Yield: | 4.8 g (43 %) | | |
| Melting point: | 87.5 - 87.8 °C | | |
| Elemental analysis: | | | |
| | C (%) | H (%) | N (%) |
| Found: | 73.66 | 7.06 | 12.27 |
| Calculated: | 73.73 | 7.07 | 12.39. |

(17) Preparation of 1-(4-ethylphenyl)-3-(1H-imidazol-1-yl)-propan-1-one (compound 17):

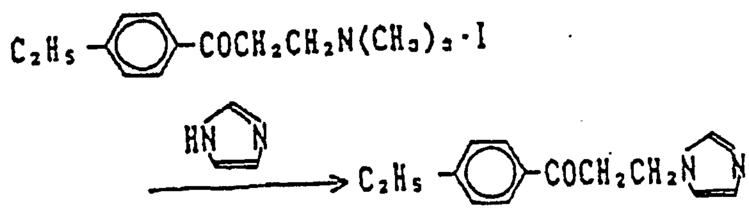

A mixture of 47.2 g (0.136 mol) of 1-(4-ethylphenyl)-3-dimethylaminopropan-1-one methoiodide, 8.4 g (0.124 mol) of imidazole and 200 ml of methanol was refluxed for 3 hours, and then the methanol was evaporated. Water was added to the residue, which was extracted with ether. Next the ether was evaporated, and the residue was purified by column chromatography.
Yellowish white crystals.

| | | | |
|---|---|---|---|
| Yield: | 5.3 g (19 %) | | |
| Melting point: | 51.6 - 57.0 °C | | |
| Elemental analysis: | | | |
| | C (%) | H (%) | N (%) |
| Found: | 73.66 | 7.06 | 12.27 |
| Calculated: | 73.21 | 7.14 | 12.00. |

(18) Preparation of 2-ethyl-1-(4-ethylphenyl)-3-(1H-imidazol-1-yl)-propan-1-one hydrochloride (compound 18):

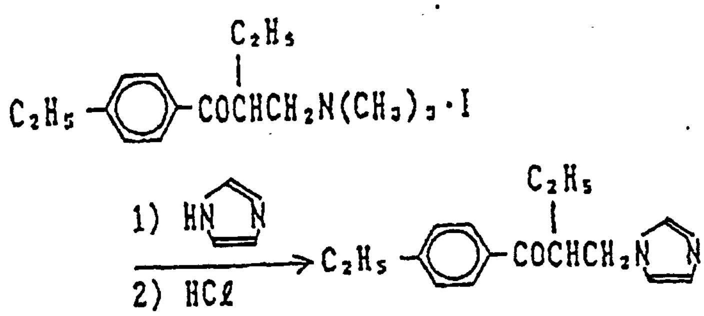


A mixture of 20.3 g (0.054 mol) of 2-ethyl-1-(4-ethylphenyl)-3-dimethylaminopropan-1-one methoiodide, 3.3 g (0.049 mol) of imidazole and 100 ml of methanol was refluxed for 3 hours, and then the methanol was evaporated. Water was added to the residue, which was extracted with ether. Next the ether was evaporated, and the residue was purified by column chromatography. Then 0.1N ethanol solution of hydrochloric acid was added to convert the reaction product into the hydrochloride.
White crystals.

| | | | |
|---|---|---|---|
| Yield: | 4.0 g (13 %) | | |
| Melting point: | 140.0 - 142.0 °C | | |
| Elemental analysis: | | | |
| | C (%) | H (%) | N (%) |
| Found: | 65.43 | 7.23 | 9.57 |
| Calculated: | 65.40 | 7.25 | 9.47. |

(19) Preparation of 1-(4-ethylphenyl)-3-(1H-imidazol-1-yl)-2-phenylpropan-1-one (compound 19):

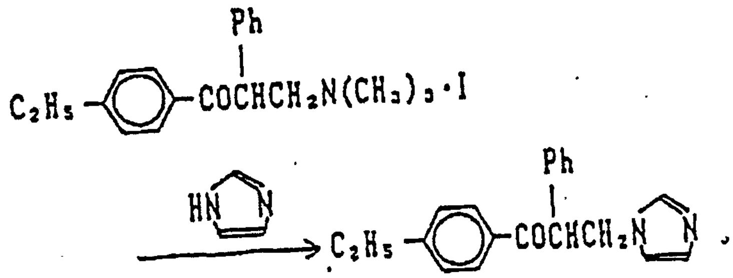

A mixture of 43.8 g (0.104 mol) of 1-(4-ethylphenyl)-3-dimethylamino-2-phenylpropan-1-one methoiodide, 6.4 g (0.094 mol) of imidazole and 100 ml of methanol was refluxed for 3 hours, and then the methanol was evaporated. Water was added to the residue, which was extracted with ether. Next the ether was evaporated, and the residue was purified by column chromatography.
White crystals.

| | | | |
|---|---|---|---|
| Yield: | 15.4 g (54 %) | | |
| Melting point: | 137.5 - 137.8 °C | | |
| Elemental analysis: | C (%) | H (%) | N (%) |
| Found: | 78,92 | 6.62 | 9.20 |
| Calculated: | 78.92 | 6.72 | 9.19. |

(20) Preparation of 1-(9H-fluoren-2-yl)-3-(1H-imidazol-1-yl)-2-methylpropan-1-one (compound 20):

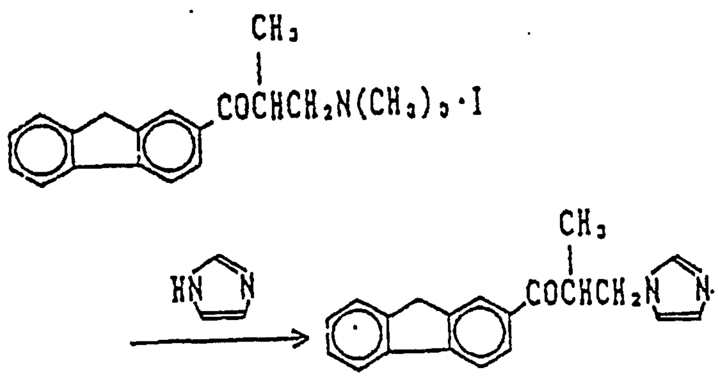

A mixture of 30.0 g (0.071 mol) of 1-(9H-fluorene-2-yl)-2-methyl-3-dimethylaminopropan-1-one methoiodide, 4.4 g (0.064 mol) of imidazole and 200 ml of methanol was refluxed for 2 days, and then the methanol was evaporated. Water was added to the residue, which was extracted with ether. Next the ether was evaporated, and the residue was purified by column chromatography.
Light yellowish white crystals.

| | | | |
|---|---|---|---|
| Yield: | 4.4 g (21 %) | | |
| Melting point: | 166.0 - 168.0 °C | | |
| Elemental analysis: | | | |
| | C (%) | H (%) | N (%) |
| Found: | 79.18 | 6.16 | 8.99 |
| Calculated: | 79.44 | 6.00 | 9.26. |

(21) Preparation of 1-(4-ethylphenyl)-2-(1H-imidazol-1-yl)-propan-1-one hydrochloride (compound 21):

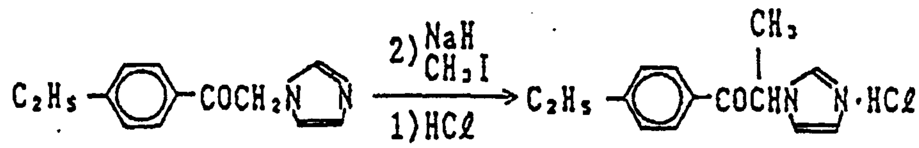


0.8 g (0.02 mol) of sodium hydride was dispersed in 15 ml of anhydrous N,N-dimethylformamide, and with stirring at 0 °C, a solution of 3.8 g (0.018 mol) of 1-(4-ethylphenyl)-2-(1H-imidazol-1-yl)-ethan-1-one dissolved in 15 ml of anhydrous N,N-dimethylformamide was slowly added. After stirring for 30 minutes at the same temperature, 1.4 ml (0.022 mol) of methyl iodide was added, and stirring was continued for 1 hour at room temperature. Next, water was added to the reaction solution, and the product was extracted with ethyl acetate. Then 0.1N ethanol solution of hydrochloric acid was added to convert the reaction product into the hydrochloride which was recrystallized from ethanol/acetone/ether mixture.
Yellowish white crystals.

| | | | |
|---|---|---|---|
| Yield: | 2.7 g (52 %) | | |
| Melting point: | 177.0 - 178.8 °C | | |
| Elemental analysis: | | | |
| | C (%) | H (%) | N (%) |
| Found: | 63.51 | 6.47 | 10.58 |
| Calculated: | 63.07 | 6.53 | 10.55. |

(22) Preparation of 1-(4-ethylphenyl)-3-(1H-imidazol-1-yl)-2-methylpropan-1-ol (compound 22):

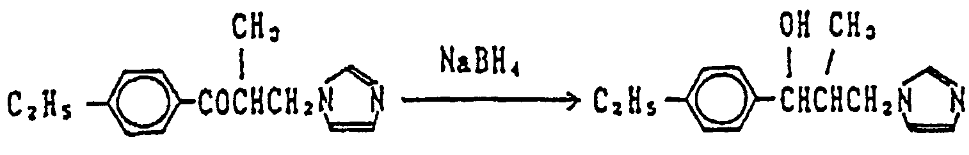


100 ml of ethanol was added to 5.0 g (0.021 mol) of 1-(4-ethylphenyl)-3-(1H-imidazol-1-yl)-2-methylpropan-1-one (compound 2); 1.2 g (0.031 mol) of sodium borohydride was further added at room temperature with stirring, and stirring was continued overnight. Next, the reaction solution was poured into water, and the product was extracted with ether. In addition, the product was crystallized by evaporation of the ether.

White crystals.

| | | | |
|---|---|---|---|
| Yield: | 4.5 g (89 %) | | |
| Melting point: | 107.0 - 110.0 °C | | |
| Elemental analysis: | | | |
| | C (%) | H (%) | N (%) |
| Found: | 73.76 | 8.32 | 11.54 |
| Calculated: | 73.74 | 8.25 | 11.47. |

(23) Preparation of 1-[[2-(4-ethylphenyl)-1,3-dioxolan-2-yl]-propyl]-1H-imidazole (compound 23):

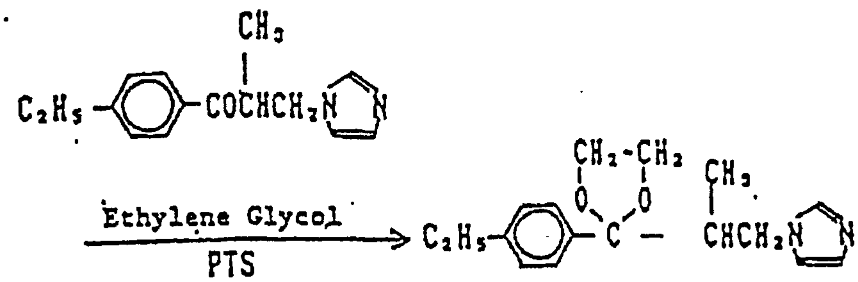


A mixture of 6.0 g (0.026 mol) of 1-(4-ethylphenyl)-3-(1H-imidazol-1-yl)-2-methylpropan-1-one (compound 2), 2.8 ml (0.051 mol) of ethylene glycol, 9.8 g (0.051 mol) of p-toluenesulfonic acid monohydrate and 100 ml of toluene was refluxed for 6 hours. After cooling, the reaction solution was poured into an alkaline aqueous solution, and the product was extracted with ethyl acetate.

Next, the ethyl acetate was evaporated, and the residue was recrystallized from ether. White crystals.

| | | | |
|---|---|---|---|
| Yield: | 4.4 g (62 %) | | |
| Melting point: | 112.0 - 114.0 °C | | |
| Elemental analysis: | | | |
| | C (%) | H (%) | N (%) |
| Found: | 71.32 | 7.74 | 9.71 |
| Calculated: | 71.30 | 7.74 | 9.78. |

(24) Preparation of 1-(4-ethylphenyl)-2-phenyl-3-(pyrrolidin-1-yl)-propan-1-one hydrochloride (compound 24):

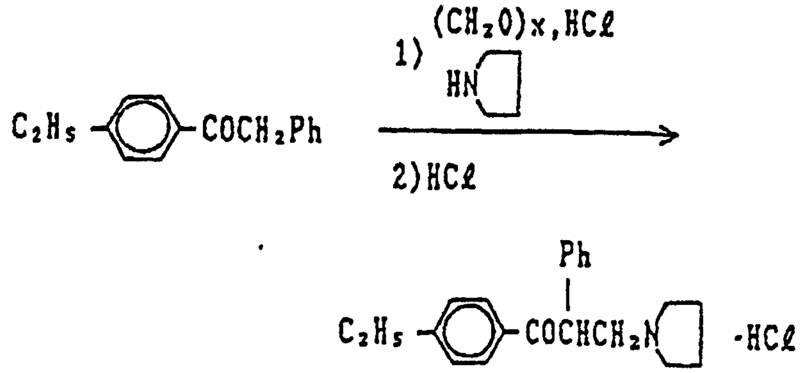


A mixture of 22.2 g (0.100 mol) of 1-(4-ethylphenyl)-2-phenylethan-1-one, 5.0 g of paraformaldehyde, 5.7 g (0.080 mol) of pyrrolidine, 10 ml of hydrochloric acid and 30 ml of isopropanol was refluxed overnight, and then the isopropanol was evaporated. Water was added to the residue, which was washed with ether, neutralized with alkali, and extracted with ether. The extract was concentrated in vacuo, and then the residue was dissolved in ethanol. 10 ml of hydrochloric acid were added to convert it into the hydrochloride which was recrystallized from ethanol/ ether mixture.
White crystals.

| | | | |
|---|---|---|---|
| Yield: | 9.5 g (33 %) | | |
| Melting point: | 155.0 - 156.5 °C | | |
| Elemental analysis: | | | |
| | C (%) | H (%) | N (%) |
| Found: | 73.83 | 7.89 | 3.91 |
| Calculated: | 73.28 | 7.63 | 3.94. |

(25) Preparation of 1-(4-chlorophenyl)-2,2-dimethyl-3-(pyrrolidin-1-yl)-propan-1-one (compound 25):

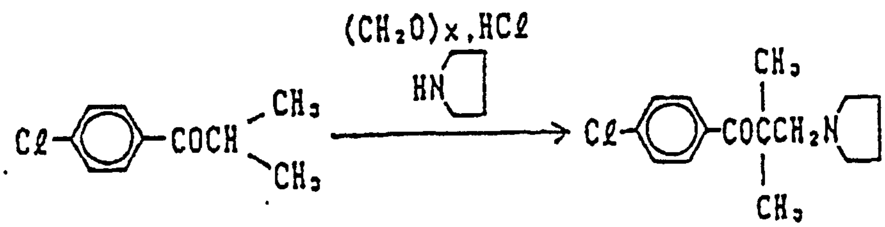


A mixture of 54.8 g (0.300 mol) of 1-(4-chlorophenyl)-2-methylpropan-1-one, 45 g of paraformaldehyde, 21.3 g (0.300 mol) of pyrrolidine and 30 ml of hydrochloric acid was stirred overnight with heating in an oil bath set to 110 °C. After cooling, the reaction solution was diluted in water, washed with ether, neutralized with alkali and extracted with ether. The ether was evaporated, and then the residue was purified by column chromatography.
Yellow oil.

| | | | |
|---|---|---|---|
| Yield: | 2.2 g (3 %) | | |
| Elementary analysis: | | | |
| | C (%) | H (%) | N (%) |
| Found: | 67,69 | 7.58 | 5.27 |
| Calculated: | 67.67 | 7.73 | 5.07. |

(26) Preparation of 1-(4-ethylphenyl)-2-methyl-3-(2-methyl-1H-imidazol-1-yl)-propan-1-one (compound 26):

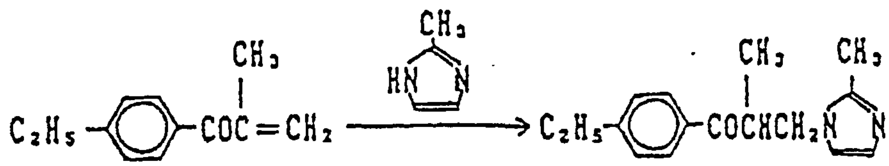

A mixture of 7.5 g (0.043 mol) of 1-(4-ethylphenyl)-2-methyl-2-propen-1-one, 3.2 g (0.039 mol) of 2-methyl-imidazole and 50 ml of methanol was refluxed overnight, and then the methanol was evaporated. Next the residue was extracted with chloroform and purified by column chromatography.
White crystals.

| | | | |
|---|---|---|---|
| Yield: | 7.1 g (64.4 %) | | |
| Melting point: | 87.0 - 89.0 °C | | |
| Elemental analysis: | | | |
| | C (%) | H (%) | N (%) |
| Found: | 75.00 | 7.94 | 10.87 |
| Calculated: | 75.00 | 7.86 | 10.93. |

(27) Preparation of 1-(4-ethylphenyl)-2-methyl-3-(1H-1,2,4-triazol-1-yl)-propan-1-one (compound 27):

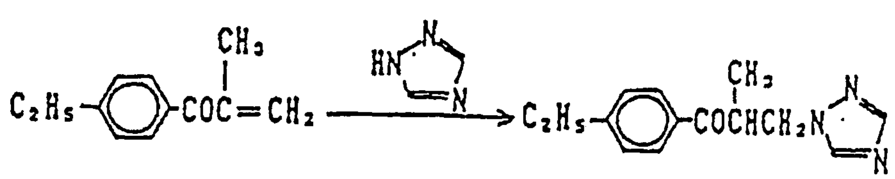

A mixture of 7.5 g (0.043 mol) of 1-(4-ethylphenyl)-2-methyl-2-propen-1-one, 2.7 g (0.039 mol) of 1,2,4-triazole and 100 ml of methanol was refluxed for 4 days, and then the methanol was evaporated. Next the residue was extracted with chloroform and purified by column chromatography.
White crystals.

| | | | |
|---|---|---|---|
| Yield: | 3.2 g (31 %) | | |
| Melting point: | 71.0 - 73.0 °C | | |
| Elemental analysis: | C (%) | H (%) | N (%) |
| Found: | 69.07 | 7.16 | 17.34 |
| Calculated: | 69.11 | 7.04 | 17.27. |

## II Pharmacological Investigations

The pharmacological properties of the compounds prepared in the above-mentioned preparation examples were determined.

(1) Inhibitory Effect on the Motor Coordination

Male Slc-ddy mice of 20 - 25 g (5 weeks old) were used in groups of 7 - 10. The test compounds were used dissolved or suspended in 0.5 % CMC or 0.5 % Tween 80 solution. Each test compound (300 - 600 mg/10 ml/kg) was orally administered to the mice, and after 30 min the mice were put on a 3-cm diameter rod rotating at a speed of 10 $min^{-1}$ in a direction opposite to the rotation direction, and the time of staying on it was measured to a maximum of 180 s. Those falling off of the rotating rod within 60 s were judged as having an inhibitory effect on the motor coordination and, with the number of cases as index, comparison was made with the reference compound (eperisone hydrochloride).

The mice were trained in advance to be able to stay on the rotating rod for more than 300 s, and those capable of staying on it for more than 250 s immediately before testing were used.

Of the test compounds found to have a stronger inhibitory effect on the motor coordination than eperisone hydrochloride, the compounds 2, 14, 15, 22 and 23 were selected. Their $ED_{50}$ values for the inhibitory effect on the motor coordination (95% confidence limit) were determined; the results are shown in Table 1. With reference to other compounds, the comparative results with eperisone hydrochloride are shown in Table 5.

### Table 1

| Eperisone hydrochloride | positive cases not in excess of 50 % |
|---|---|
| Compound 2 | 200.0 mg/kg (153.2-261.1) |
| Compound 14 | 100.0 mg/kg ( 69.0-144.8) |
| Compound 15 | 152.0 mg/kg (116.7-197.9) |
| Compound 23 | 210.0 mg/kg (153.6-287.0) |

(2) Anti-nicotinic Effect

Nicotine is known to stimulate the central nervous system and the skeletal muscles. When administered to some animals, convulsions and dyspnea are caused to eventually kill the animal. If the nicotinic effect can be suppressed by administration of any of the above-mentioned compounds, this compound is expected to have a central muscle relaxation effect.

Experimental Method of Male Slc-ddy mice/30 - 35 g (6 weeks old) were used as test animals in groups

of 7 - 10. The test compounds (100 mg/10 ml/kg) were orally administered to the mice, and after 30 min, nicotine bitartrate (3 mg/10 ml/kg) was administered intravenously, and the number of animals killed due to tonic convulsion was counted. The surviving animals were judged as having an anti-nicotinic effect, and with the number thereof as index the comparison concerning the anti-nicotinic effect was made with the reference compound (eperisone hydrochloride). Of the test compounds found to have a stronger anti-nicotinic effect than eperisone hydrochloride, the compounds 2, 14 and 23 were selected. The $ED_{50}$ values (95 % confidence limit) for the anti-nicotinic effect thereof were determined by the Litchfield-Wilcoxon method and are shown in Table 2. With reference to other compounds, the comparative results with eperisone hydrochloride are shown in Table 5.

**Table 2**

| | |
|---|---|
| Eperisone hydrochloride | 140.0 mg/kg (95.4 - 205.5) |
| Compound 2 | 26.0 mg/kg (13.4 - 50.3) |
| Compound 14 | 32.0 mg/kg (18.5 - 55.4) |
| Compound 23 | 46.0 mg/kg (23.9 - 88.6) |

(3) Anti-strychnine Effect

Strychnine is known to enhance reflex stimulation, among others, of spinal cord to induce convulsions. If this effect of strychnine can be suppressed by administration of any of the above-mentioned compounds, this compound is expected to have a central muscle relaxation effect.

Experimental Method

Male Slc-ddy mice of 25 - 30 g (5 weeks old) were used in groups of 7 - 10.

The test compounds (100 mg/10 ml/kg) were orally administered to the mice, and after 30 min, 1.5 mg/10 ml/kg of strychnine nitrate was administered subcutaneously, and the time was measured from the occurence of tonic convulsions to the death of the animals. Cases in which this time was extended to 6 min or more were judged as corresponding to an anti-strychnine effect, and with the number of such cases as index, comparison was made with the reference compound (eperisone hydrochloride). Of the test compounds found to have a stronger anti-strychnine effect than eperisone hydrochloride, the compounds 2, 14 and 23 were selected. The $ED_{50}$ values (95% confidence limit) for the anti-strychnine effect were determined by the Litchfield-Wilcoxon method and are shown in Table 3. With reference to other compounds, the comparative results with eperisone hydrochloride are shown in Table 5.

**Table 3**

| | |
|---|---|
| Eperisone hydrochloride | No effect detected |
| Compound 2 | 135.0 mg/kg ( 87.2-209.0) |
| Compound 14 | 33.0 mg/kg ( 19.0- 57.3) |
| Compound 23 | 155.0 mg/kg (116.9-205.6) |

(4) Acute Toxicity Test

Male ddy mice of 20-30 g were used as test animals in groups of 5-10. The test compounds were dissolved or suspended in 0.5 % Tween 80 aqueous solution, and orally administered to the mice. Observation was made for 7 days after administration. The $LD_{50}$ values (50% of lethal dose) (95% confidence limit) were determined by the Litchfield-Wilconxon method, and are shown in Table 4.

**Table 4**

| | |
|---|---|
| Eperisone hydrochloride | 303 mg/kg (259-355) |
| Compound 2 | 218 mg/kg (178-267) |
| Hydrochloride of compound 2 | 225 mg/kg (182-278) |
| Compound 14 | 209 mg/kg (179-244) |
| Compound 23 | 415 mg/kg (330-522) |

Table 5

| Compound No. | Inhibitory effect on motor coordination | | Anti-nicotinic effect | | Anti-strychnine effect |
|---|---|---|---|---|---|
| | Effect | Durability | Effect | Durability | |
| Compound 1 | 0 | - | - | | |
| Compound 2 | 0 | X | 0 | X | 0 |
| Compound 3 | 0 | X | 0 | X | |
| Compound 4 | 0 | - | 0 | | |
| Compound 5 | 0 | - | 0 | | |
| Compound 6 | 0 | X | 0 | X | - |
| Compound 7 | 0 | - | 0 | | |
| Compound 8 | 0 | X | - | | |
| Compound 9 | 0 | X | 0 | | |
| Compound 10 | 0 | X | 0 | | |
| Compound 11 | 0 | X | - | | 0 |
| Compound 12 | - | - | 0 | | |
| Compound 13 | 0 | - | 0 | | |
| Compound 14 | 0 | X | 0 | X | 0 |
| Compound 15 | 0 | - | 0 | | 0 |

Table 5 (Continued)

| Compound No. | Inhibitory effect on motor coordination | | Anti-nicotinic effect | | Anti-strychnine effect |
|---|---|---|---|---|---|
| | Effect | Durability | Effect | Durability | Effect |
| Compound 16 | 0 | X | 0 | | |
| Compound 17 | 0 | - | 0 | | |
| Compound 18 | 0 | X | 0 | X | - |
| Compound 19 | 0 | - | 0 | X | |
| Compound 20 | 0 | - | 0 | X | 0 |
| Compound 21 | - | - | 0 | | |
| Compound 22 | 0 | X | 0 | | 0 |
| Compound 23 | 0 | X | 0 | | 0 |
| Compound 24 | 0 | X | 0 | | |
| Compound 25 | 0 | X | 0 | | - |
| Compound 26 | 0 | - | 0 | | |
| Compound 27 | 0 | - | 0 | | |

In the columns of effect in Fig. 5, the compounds having a stronger effect than eperisone hydrochloride are indicated by "0", those having an effect equal to eperisone hydrochloride are indicated by "0", and those with which no effect was recognizable or the effect recognized was weaker than that of eperisone hydrochloride are indicated by "-".

In the column of durability, the compounds having a durability are indicated by

"X",

and those having no durability are indicated by "-".

The results obtained show that with the test compounds, pharmacological central muscle relaxant and the anti-convulsive effects may be obtained which are generally stronger than that of eperisone hydrochloride, and durability may be achieved. Further, an anti-strychnine effect which eperisone hydrochloride doesn't have appears. Further, for eperisone hydrochloride, the minimum lethal dose is close to the minimum amount necessary for developing a pharmacological effect. On the other hand, many test compounds show that the minimum lethal dose is apart from the minimum amount for the development of a pharmacological effect. This means that these central muscle relaxants and anticonvulsives have a wide safety margin for use.

## Claims

1. Compounds represented by the general formula I

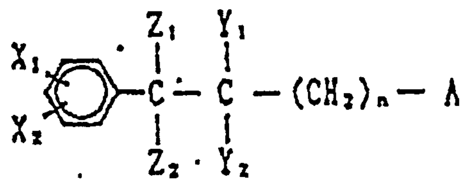 (I),

in which

$X_1$ ,$X_2$, being the same or different, represent a hydrogen atom, $C_{1-4}$-alkyl, phenyl, halogen, $C_{1-4}$-alkoxy or dialkylamino with $C_{1-4}$-alkyl groups, or $X_1$ and $X_2$ together represent phenylene or indane-2,3-diyl,

$Z_1$, $Z_2$, being the same or different, represent a hydrogen atom or hydroxy, or together represent oxygen or $-O(CH_2)_2O-$,

$Y_1$, $Y_2$, being the same or different, represent a hydrogen atom, $C_{1-3}$-alkyl or phenyl,

A represents a group derived from a cyclic amine,

and

n represents 0, 1 or 2,

in the form of the free base or in the form of addition salts, preferably pharmaceutically acceptable salts.

2. Compounds of the general formula I according to claim 1, wherein $X_1$ and $X_2$, being the same or different, represent hydrogen, $C_{2-4}$-alkyl or halogen, or together represent phenylene.

3. Compounds of the general formula I according to claim 1 or 2, wherein $X_1$ represents 4-ethyl, and $X_2$ represents a hydrogen atom.

4. Compounds of the general formula I according to claim 1 or 2, wherein $X_1$ and $X_2$ together represent 3,4-dimethyl.

5. Compounds of the general formula I according to claim 1 or 2, wherein $X_1$ and $X_2$ together represent phenylene.

6. Compounds of the general formula I according to claims 1 to 5, wherein one of $Y_1$ and $Y_2$ represents methyl, and the other represents a hydrogen atom.

7. Compounds of the general formula I according to claims 1 to 6, wherein A represents piperidino, pyrrolidine-1-yl or imidazole-1-yl.

8. Compounds of the general formula I according to claims 1 to 7, wherein n is equal to 1, and/or A represents imidazole-1-yl.

9. Compounds of the general formula I, wherein $Z_1$ and $Z_2$ together represent an oxygen atom, and one of $Y_1$ and $Y_2$ represents methyl, and the other is hydrogen.

10. Compounds of the formulae

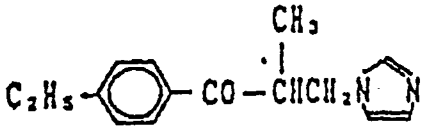

(II),

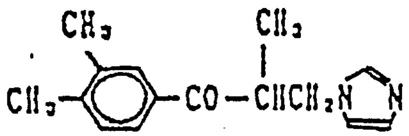

(III),

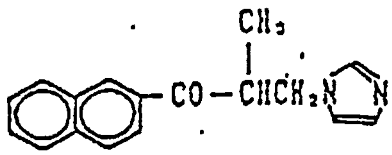

(IV),

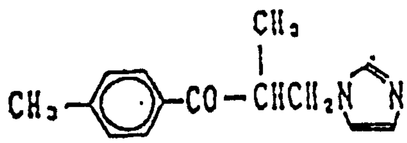

(V),

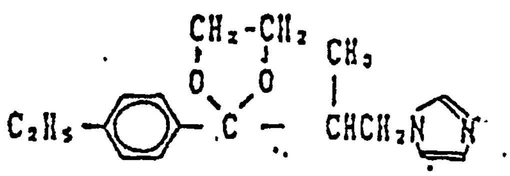

(VI),

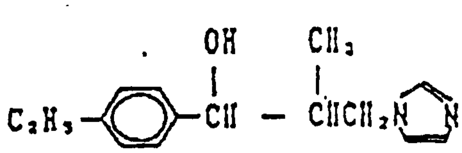

(VII),

and

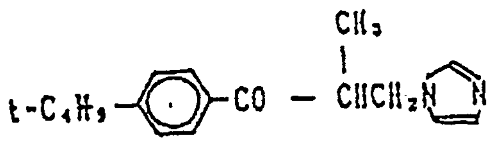

(VIII).

**11.** Method for preparing the compounds represented by the general formula I according to claims 1 to 10, characterized by the following measures:

(A) for preparing compounds of the general formula X,

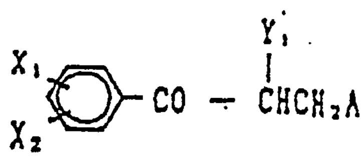

(X),

wherein $X_1$, $X_2$, $Y_1$ and A are defined as in claim 1, a compound of the general formula IX,

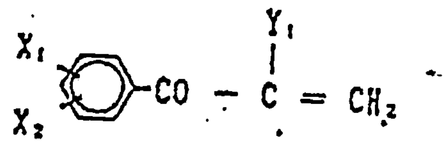

(IX),

wherein $X_1$, $X_2$ and $Y_1$ are defined as in claim 1,

is caused to react with a cyclic amine or a reactive derivative thereof;

(B) for preparing compounds of the general formula XII,

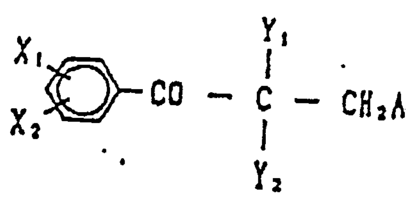

(XII),

wherein $X_1$, $X_2$, $Y_1$, $Y_2$ and A are defined as in claim 1,

a compound of the general formula XI,

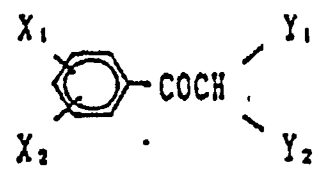 (XI),

wherein $X_1$, $X_2$, $Y_1$ and $Y_2$ are defined as in claim 1, is caused to react with paraformaldehyde or formalin and the respective cyclic amine or a reactive derivative thereof or a mineral acid salt thereof, particularly the hydrochloride;

(C) for preparing compounds of the general formula XIV,

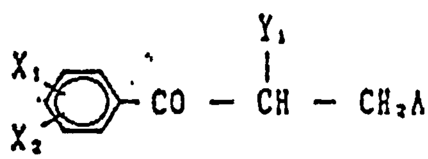 (XIV),

wherein $X_1$, $X_2$, $Y_1$ and A are defined as in claim 1,

a compound of the general formula XIII,

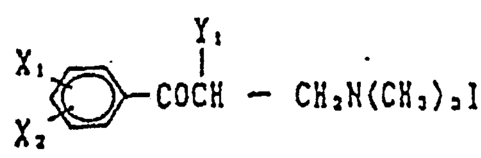 (XIII),

wherein $X_1$, $X_2$ and $Y_1$ are defined as in claim 1,

is caused to react with a cyclic amine or a reactive derivative thereof;

(D) for preparing compounds of the general formula XVI,

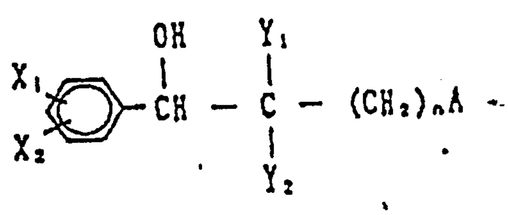 (XVI),

wherein $X_1$, $X_2$, $Y_1$, $Y_2$, A and n are defined as in claim 1,

a compound of the general formula XV,

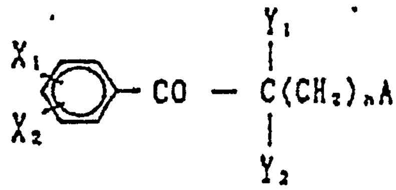

(XV),

wherein $X_1$, $X_2$, $Y_1$, $Y_2$, A and n are defined as in claim 1,

is reduced by means of a reducing agent;
(E) for preparing compounds of the general formula XVII,

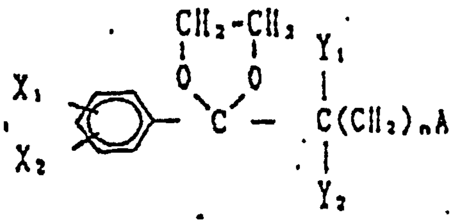

(XVII),

wherein $X_1$, $X_2$, $Y_1$, $Y_2$, A and n are defined as in claim 1, a compound of the above-defined formula XV

is caused to react with ethylene glycol;
(F) for preparing compounds of the general formula XIX,

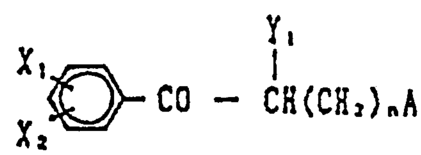

(XIX),

wherein $X_1$, $X_2$, $Y_1$, A and n are defined as in claim 1,

a compound of the general formula XVIII,

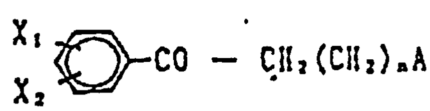

(XVIII),

wherein $X_1$, $X_2$, A and n are defined as in claim 1,

is caused to react with sodium hydride and an alkyl halide.

**12.** Pharmaceutical compositions, preferably having central muscle relaxant and/or anticonvulsive activity, characterized in that they comprise one or more of the compounds of the formulae I to VIII according to claims 1 to 10 as active ingredients, salts thereof being pharmaceutically acceptable.

**13.** Use of the compounds of the formulae I to VIII according to claims 1 to 10 for preparing pharmaceutical compositions, particularly for muscle relaxation and/or anticonvulsive treatment of mammalians.